# EUROPEAN PATENT SPECIFICATION

(11) **EP 2 566 552 B1**
(45) Date of publication and mention of the grant of the patent: **05.04.2017**
(21) Application number: 10720681.5
(22) Date of filing: 07.05.2010
(51) Int. Cl.: A61M 16/00, G06F 3/0488, G06F 3/0484, G06F 19/00

(54) **BREATHING APPARATUS WITH USER INTERFACE**
BEATMUNGSVORRICHTUNG MIT BENUTZEROBERFLÄCHE
APPAREIL RESPIRATOIRE AVEC INTERFACE UTILISATEUR

(43) Date of publication of application: 13.03.2013
(73) Proprietor: Maquet Critical Care AB, 171 95 Solna (SE)
(72) Inventor: FOGELBRINK, Petter, 194 51 Upplands Väsby (SE)
(74) Representative: Zacco Sweden AB
(86) International application number: PCT/SE2010/050505
(87) International publication number: WO 2011/139194

(56) References cited:
- EP-A1- 0 901 798
- EP-A2- 2 169 528
- WO-A1-99/47200
- WO-A1-2009/005957
- DE-A1-102008 032 453
- US-A1- 2008 072 896
- US-B1- 6 476 797

## Description

### TECHNICAL FIELD

The present invention relates to a breathing apparatus having a user interface comprising a contact-sensitive screen through which a breathing apparatus setting can be changed, according to the preamble of claim 1.

### BACKGROUND

Contact-sensitive screens, or touch screens, are often used in breathing apparatuses, such as ventilators, in order to provide a user interface through which an operator can interact with the breathing apparatus. When the operator wants to change a breathing apparatus setting related to the operation of the breathing apparatus, the touch screen is normally used only to select the parameter that is to be set, and, sometimes, to confirm the new setting of the parameter. The act of changing the setting, however, is normally performed by rotating a rotary knob, pressing a +/button, or sliding a slide button, which knob or button is located external to the screen on the breathing apparatus body.

Thus, a typical chain of actions for changing a breathing apparatus setting comprises the steps of:
- selecting the setting to be changed by touching a touch area associated with the setting on the touch screen, typically realized as a button carrying a name of the setting or the parameter to which the setting relates;
- rotating a rotary knob or pressing/sliding a button on the breathing apparatus body to change the setting to a desired value, and
- confirming the new setting by touching another touch area on the touch screen, typically realized as a "confirm" or an "accept" button, or by pressing the rotary knob.

A ventilator in which ventilator settings are changed in accordance with the above principle is disclosed in US patent No. 5,678,539.

EP 0901798 discloses a breathing apparatus comprising a user interface for programming the breathing apparatus's operating mode, comprising an interactive screen with a touch-sensitive surface capable. Programming of the breathing apparatus, e.g. changing a parameter setting, can be performed by an operator via the user interface by touching specially arranged segments on the screen one or more times. The user interface further comprises a verification switch for implementing active storage in the breathing apparatus of indicated programming.

WO 99/47200 discloses a medical ventilator having a user interface. The user interface includes a touch screen having different touch areas through which various ventilator settings can be changed by the touch of an operator.

In US 2008/072896 it is described that a graphic user interface for a breathing assistance system may provide different views, where each view may provide different levels of access to breathing assistance/ventilation parameters. Some of these views may have restricted access in order to prevent an unauthorized person to get access to a view with a higher level of access to breathing assistance parameters than intended. In order to get access to a restricted view, a user may be required to perform a certain action. One example of such an action mentioned in US 2008/072896 is to touch icons simultaneously.

Although not commonly used in the art, there are also user interfaces allowing an operator to change breathing apparatus settings using nothing but a touch screen of the breathing apparatus. This is advantageous in that the entire process can be performed using one and the same device. However, compared to the two-device solution requiring a breathing apparatus operator to use both the touch screen and e.g. a rotary knob of the apparatus in order to change breathing apparatus settings, the risk for unintentional changes of breathing apparatus settings is increased. Unintentional changes of critical breathing apparatus settings may have undesired effects on the ongoing respiratory treatment provided to a patient by the breathing apparatus, and even pose a risk to the safety of the patient.

### SUMMARY

It is an object of the invention to provide a Human-Machine Interface (HMI) for a breathing apparatus offering a simple but yet safe way of changing breathing apparatus settings.

This is achieved by a breathing apparatus according to claim 1. The breathing apparatus according to the invention has a user interface comprising a contact-sensitive screen through which a user can change breathing apparatus settings relating to the operation of the breathing apparatus. The contact-sensitive screen comprises a first touch area through which a breathing apparatus setting can be changed by the touch of a user, and at least a second touch area at a distance from said first touch area. The user interface is configured such that the breathing apparatus setting can only be changed by touching said first and second touch areas simultaneously.

Thus, the invention presents a breathing apparatus having a user interface comprising a contact-sensitive screen, i.e. a touch screen, through which a breathing apparatus setting can be changed. To avoid unintentional changes of the settings and hence unintentional changes in the operation of the breathing apparatus, the user interface is configured such that a two-point contact with the touch screen is required to change the breathing apparatus setting. Thereby, the breathing apparatus setting and the operation of the breathing apparatus can be changed by an operator in a simple and safe way by interacting with nothing but the contact-sensitive screen, i.e. without having to interact with devices such as rotary knobs or buttons which are arranged external to the contact-sensitive screen on the breathing apparatus body.

The first touch area comprises contact-sensitive control means through which the user can change a breathing apparatus setting only if simultaneously touching the second touch area. The second touch area can hence be said to form a "dead man's handle" which has to be touched in order for the operator to change a breathing apparatus setting through the first touch area. Thereby, the first and second touch areas form a two-point touch control through which breathing apparatus settings can be changed. Since the first and second touch areas are separate areas located at a distance from each other on the contact-sensitive screen, the risk of unintentionally changing the breathing apparatus setting becomes small. The distance between the first and second touch areas is at least 1 cm, and preferably at least 2 cm, to minimize the risk that the operator makes simultaneous contact with both the first and the second touch areas if unintentionally touching the contact-sensitive screen with a finger. That the distance between the first touch area and the second touch area should be at least 1 cm means that there should be at least 1 cm to the closest point of the second touch area from any point of the first touch area.

A touch area should herein be interpreted as an area of a contact-sensitive screen within which a touch by a user can be detected by the device of which the screen forms a part.

In some embodiments, the first and second touch areas are located at a distance from each other which is sufficiently short to allow an operator of the breathing apparatus to touch the first and second touch areas simultaneously using only one hand. To this end, the distance from any point of the first touch area to the closest point of the second touch area should preferably be within the range of 1-15 cm, and even more preferably within the range of 1-5 cm. In these embodiments, the first and second touch areas form a two-point touch control intended for one-handed operation.

In other embodiments, the first and second touch areas are located at a longer distance from each other requiring the operator to use two hands in order to change breathing apparatus settings. In these embodiments, the first and second touch areas form a two-point touch control intended for two-handed operation.

In one embodiment of a two-point touch control intended for one-handed operation, the first touch area is realized as a contact-sensitive slide control which is curved and located in relation to the second touch area such that a finger can be moved along the slide control while simultaneously contacting the second touch area with the thumb during a rotary motion of a hand.

In another embodiment of a two-point touch control intended for one-handed operation in which the first touch area is realized as a contact-sensitive slide control, both the slide control and second touch area are substantially straight, elongated areas running in parallel at a short distance from each other, such that the operator can change the breathing apparatus setting by simultaneously sliding two fingers of the same hand up and down the slide control and the second touch area.

According to another embodiment of the invention, the contact-sensitive screen comprises a plurality of second touch areas, each associated with a particular breathing apparatus setting, and a single first touch area comprising a contact-sensitive control means, such as a contact-sensitive slide control, contact-sensitive +/- buttons, or a contact-sensitive rotary knob, through which any of the plurality of breathing apparatus settings can be changed by touching the second touch area associated with that particular setting while manipulating the contact-sensitive control means of the first touch area.

According to yet another embodiment of the invention, the contact-sensitive screen comprises a plurality of first touch areas, each associated with a certain breathing apparatus setting, and a single second touch area which, when touched, allows the breathing apparatus setting associated with any of the plurality of first touch areas to be changed. The second touch area may be located close to an edge of the contact-sensitive screen to allow the operator to make continuous contact with the second touch area while resting his hand against a supporting surface external to the screen, such as a frame of a display unit of which the screen forms a part. In this way, the contact-sensitive screen is "unlocked" by touching (and holding) the second touch area, whereupon the various breathing apparatus settings can be changed through the respective first touch areas.

Another object of the invention is to provide a breathing apparatus the operation of which may be changed in a simple but yet safe manner.

This object is achieved by a breathing apparatus according to claim 1. The breathing apparatus comprises a display unit of which the contact-sensitive screen forms a part, and a display control unit for receiving and processing data input by an operator on the contact-sensitive screen, and for causing information to be displayed on the screen in response thereto. The display control unit is configured to determine whether the first and second touch areas are touched or not, and to change the breathing apparatus setting in response to a touch of the first touch area only if the second touch area is touched simultaneously.

Herein described is a method offering a simple but yet safe way of changing the operation of a breathing apparatus.

Herein described is a method for changing a breathing apparatus setting affecting the operation of a breathing apparatus, comprising the steps of:
- displaying, on a contact-sensitive screen, a first touch area through which said breathing apparatus setting can be changed by the touch of the operator;
- displaying, on said contact-sensitive screen, a second touch area at a distance from said first touch area,
- determining whether said first and second touch areas are touched or not, and
- changing the breathing apparatus setting only in response to simultaneous touches of the first and second touch areas.

Further advantageous features of the breathing apparatus of the invention will be described in the detailed description following hereinafter.

### BRIEF DESCRIPTION OF THE DRAWINGS

Fig. 1 illustrates a breathing apparatus according to an exemplary embodiment of the invention.
Fig. 2 illustrates a user interface for a breathing apparatus according to an exemplary embodiment of the invention.
Fig. 3 illustrates a user interface for a breathing apparatus according to another exemplary embodiment of the invention.
Fig. 4 illustrates a user interface for a breathing apparatus according to another exemplary embodiment of the invention.
Fig. 5 illustrates a user interface for a breathing apparatus according to yet another exemplary embodiment of the invention.

### DETAILED DESCRIPTION

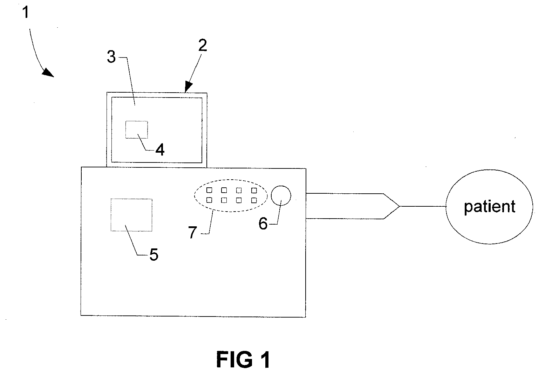

Fig. 1 illustrates a breathing apparatus 1 comprising a display unit 2 having a contact-sensitive screen 3 through which a breathing apparatus operator can change breathing apparatus settings affecting the operation of the breathing apparatus, thereby adjusting the respiratory treatment of a patient connected to the breathing apparatus. The breathing apparatus 1 may be a ventilator, an anaesthesia apparatus or any other breathing apparatus configured to provide respiratory care to a patient through the supply of breathing gas.

The contact-sensitive screen 3 is a multi-touch screen providing a user with the ability to apply multiple finger gestures (hereinafter referred to as "touches") simultaneously onto the screen to send commands to the breathing apparatus 1. The display unit 2 comprises a display control unit 4 for receiving and processing data input by an operator on the contact-sensitive screen 3, and for causing information to be displayed on the screen in response thereto. The display control unit 4 is further operable to cause a change in the operation of the breathing apparatus 1 based on information input by the operator through the screen 3. The display control unit 4 may to this end be connected to a central control unit 5 of the breathing apparatus, which is configured to effectuate the change in operation of the breathing apparatus based on the information input by the operator. For example, the central control unit 5 may be configured to control different valves in the breathing apparatus 1 to alter the flow rate or pressure with which the breathing gas is supplied to the patient, the composition of the breathing gas supplied to the patient, the positive end-expiratory pressure (PEEP) applied to the patient by the breathing apparatus, etc., in response to signals received from the display control unit 4.

In addition to the contact-sensitive screen 3 of the display unit 2, the breathing apparatus 1 may comprise other means through which the operator can change breathing apparatus settings. For example, the breathing apparatus may comprise a rotary knob 6 and/or buttons 7, arranged external to the display unit 3 on the breathing apparatus body.

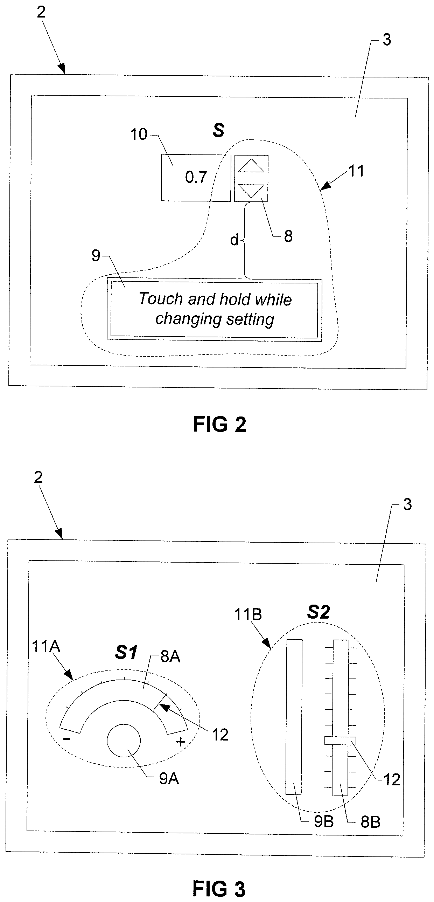

Fig. 2 illustrates an exemplary view of a user interface for the breathing apparatus 1 shown in Fig. 1, as presented on the contact-sensitive screen 3 of the display unit 2. In this view, the screen 3 comprises a first 8 and a second 9 touch area, located at a distance d from each other, through which the operator can change a breathing apparatus setting denoted *S*. The screen 3 also comprises a current setting indication area 10 in which the current value of the breathing apparatus setting *S* is displayed. In this exemplary view, the setting *S* is the setting of a parameter having a current value of 0,7.

The first touch area 8 comprises two arrows forming contact-sensitive control means through which the setting *S* can be changed. However, in order to avoid unintentional changes of the setting *S*, it can only be changed by touching the first touch area 8 while simultaneous contact is made with the second touch area 9, i.e. if the operator touches the first and second touch areas simultaneously. The first 8 and second 9 touch areas can hence be said to form a "two-point touch control" 11 for the setting *S*, and the second touch area 9 can be said to serve as a "dead man's handle" which has to activated (i.e. touched) in order for the first touch area 8 to function as intended.

The distance d should be long enough to prevent the operator from unintentionally making simultaneous contact with the first 8 and second 9 touch areas. According to the invention, the distance d between the two touch areas 8, 9 should be at least 1 cm, corresponding approximately to the width of a finger tip.

With simultaneous reference to Figs. 1 and 2, the display control unit 3 may be configured to keep track of whether the second touch area 9 is activated (i.e. touched) or not, and to effectuate a change in operation of the breathing apparatus 1 based on a signal received in response to a touch of the first touch area 8 only if the second touch area 9 is activated when receiving the signal.

Fig. 3 illustrates another exemplary view of the user interface for the breathing apparatus 1 shown in Fig. 1, as presented on the contact-sensitive screen 3 of the display unit 2. In this view, two different breathing apparatus settings *S1* and *S2* can be changed by the operator through a respective two-point touch control 11A, 11 B.

The first two-point touch control 11A comprises a first touch area 8A and a second touch area 9A which, as described above with reference to Fig. 2, have to be touched simultaneously by the operator in order to change the breathing apparatus setting *S1*. In this embodiment, the first touch area 8A comprises a contact-sensitive control means in form of a contact-sensitive slide control allowing the operator to change the setting *S1* by sliding a finger in different directions on the first touch area 8A while simultaneously making contact with the second touch area 9A. The operator can increase the value of *S1* by sliding the finger towards a plus sign located at one end of the slide control, and decrease the value of *S1* by sliding the finger towards a minus sign at the other end of the slide control. To indicate the current setting to the operator, the first touch area 8A comprises a current setting indicator 12 which is movable along the slide control in response to the slide of a finger on the slide control. The user interface may be realized such that the colour of the slide control is different on different sides of the current setting indicator 12, and such that the front between the different colours is moved back and forth over the slide control in response to the sliding movement of the operator's finger. In this way, the operator receives a clear visual indication of the change in the setting *S1* resulting from the slide of the finger on the slide control.

The slide control is curved and located in relation to the second touch area 9A such that a finger can be moved along the slide control through a rotary motion of a hand while simultaneously touching the second contact 9A area with the thumb of the same hand. To this end, the elongated slide control is preferably concave in the direction of the second touch area 9A. For example, the slide control may have the shape of a circumferential segment of a circle, and the second touch area 9A may be formed as a button located in or close to the centre of said circle.

The second two-point touch control 11 B comprises a first touch area 8B and a second touch area 9B which have to be touched simultaneously by the operator in order to change the breathing apparatus setting *S2*. In this embodiment, the first touch area 8B comprises a contact-sensitive control means in form of an elongated and substantially straight contact-sensitive slide control. To indicate the current setting to the operator, the slide control comprises a current setting indicator 12 which is movable along the slide control in response to a slide of a finger. The second touch area 9B is formed as an elongated area running alongside said slide control, at a distance therefrom. Thus, the first 9A and second contact 9B areas are formed as two elongated areas which are arranged in parallel in their longitudinal directions. This configuration of the two-point touch control 11 B allows the operator to change the setting *S2* by sliding two fingers up and down the first 8B and second 9B touch area. Preferably, the distance between the first 8B and second 9B touch areas, in a direction perpendicular to their longitudinal directions, should be short enough to allow the operator to change the setting *S2* by means of a sliding movement of a single hand. Even more preferably, the distance should be short enough to allow the operator to change the setting *S2* by using two adjacent fingers of a hand, meaning that the distance should be between 1 and 10 cm, and preferably between 1 and 5 cm.

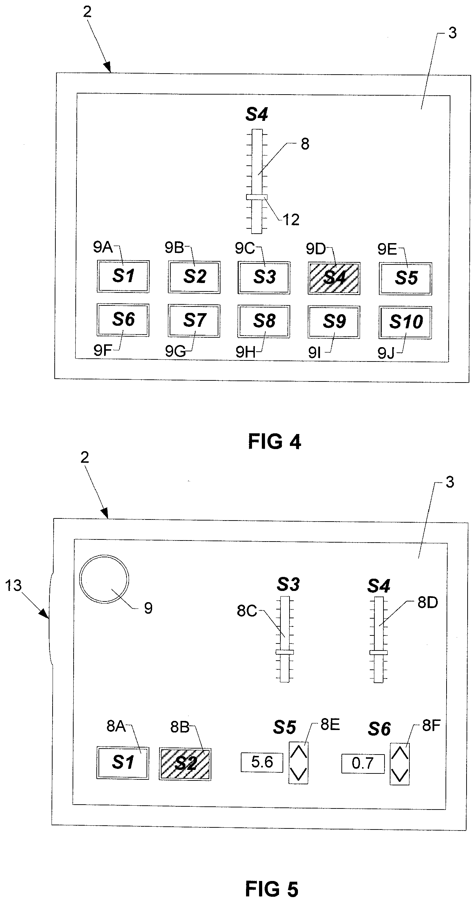

Fig. 4 illustrates another exemplary view of the user interface for the breathing apparatus 1 shown in Fig. 1, as presented on the contact-sensitive screen 3 of the display unit 2. In this view, the contact-sensitive screen 3 comprises a plurality (i.e. more than one) of second touch areas 9A-9J, each associated with a certain breathing apparatus setting *S1-S10,* and a single first touch area 8 allowing the breathing apparatus operator to change the any of the settings *S1-S10* by touching the second touch area 9A-9J associated with that setting, and said single first touch area 8, simultaneously. The first touch area 8 comprises contact-sensitive control means which, in this exemplary case, is realized in form of an elongated and substantially straight slide control as described above with reference to Fig. 3. In this embodiment, the contact-sensitive screen 3 can hence be said to comprise a plurality of two-point touch controls comprising a respective second touch area 9A-9J and a first touch area 8 which is common for all two-point touch controls.

When the operator selects a breathing apparatus setting *S1-S10* by touching the second touch area 9A-9J associated with that setting, the visual appearance of the second touch area 9A-9J that is touched may be changed to visually indicate the selection to the operator, e.g. by changing the colour thereof. Also, information indicative of the selected breathing apparatus setting may be displayed in or close to the first touch area 8 so as to clearly indicate to the operator which setting may be changed by manipulating the contact-sensitive control means of the first touch area 8 while touching the selected second touch area. Furthermore, when a breathing apparatus setting *S1-S10* is selected by the operator by touching one of the second touch areas 9A-9J, the current setting indicator 12 of the slide control may be automatically adjusted to indicate the current value of the selected setting. In the illustrated scenario, the operator has touched, or is touching, the second touch area 9D associated with a breathing apparatus setting denoted *S4*, the value of which is indicated through the position of the current setting indicator 12.

In the embodiments of the user interface described with reference to Figs. 2 and 3, the second touch areas 9, 9A-B are "passive" areas in the sense that they serve no purpose other than preventing the operator from unintentionally changing a breathing apparatus setting. In this embodiment, however, the second touch areas 9A-9J serve the double purpose of preventing the operator from unintentionally changing a breathing apparatus setting, and selecting one of a plurality of settings *S1-S10,* the value of which is to be displayed and/or changed.

Since the distance between the first touch area 8 and some of the second touch areas 9A-9J may be too long in order for the operator to touch the first touch area 8 and the second touch area 9A-9J simultaneously using fingers of the same hand, changing all or some of the settings *S1-S10* may require a two-hand operation by the operator.

Fig. 5 illustrates yet another exemplary view of the user interface for the breathing apparatus 1 shown in Fig. 1, as presented on the contact-sensitive screen 3 of the display unit 2. In this view, the contact-sensitive screen 3 comprises a plurality of first touch areas 8A-8F, each associated with a certain breathing apparatus setting *S1-S6*, and a single second touch area 9 allowing the breathing apparatus operator to change any of the breathing apparatus settings by touching the first touch area 8A-8F associated with that setting, and said single second touch area 9, simultaneously. If the second touch area 9 is not touched, none of the breathing apparatus settings *S1-S6* can be changed by touching the first touch areas 8A-8J. If, however, the second touch area 9 is touched (and held), any of the breathing apparatus settings *S1-S6* can be changed via the first touch areas 8A-8F. Thus, in this embodiment, the touch and hold of the second touch area 9 serves to "unlock" the contact-sensitive screen 3 such that the breathing apparatus settings *S1-S6* can be changed.

In this embodiment, the breathing apparatus settings denoted *S3-S6* are settings allowing the operator to change different parameter values, such as the PEEP, the oxygen concentration in the breathing gas etc, while the breathing apparatus settings denoted *S1-S2* are settings allowing the operator to switch between different modes of ventilation, e.g. between volume-controlled and pressure-controlled ventilation of a patient. According to another exemplary embodiment, the settings denoted *S1-S2* are settings allowing the operator to automatically adapt the operation of the breathing apparatus to different patient categories. The first touch areas denoted 8C-8F comprise a respective contact-sensitive control means in form of a slide-control or set of buttons through which the parameter settings *S3-S6* can be changed. The first touch areas denoted 8A-8B, however, comprises contact-sensitive control means in form of a respective button which can be touched by the operator while simultaneously touching the second touch area 9 in order to select a preferred mode of ventilation.

The second touch area 9 is located in the upper left part of the contact-sensitive screen 3 at a location allowing the operator to touch it using his thumb while resting his hand against the frame of the display unit 2. To indicate the intended position of the operator's hand while interacting with the screen 3, the frame of the display unit 2 comprises a hand position indicator 13 which, for example, may be a convexity formed on the frame of the display unit 2, against which the operator may rest the palm of his left hand while touching the second touch area 9 with the thumb of the same hand. In this embodiment, the user interface is adapted for a right-handed person. However, the contact-sensitive screen 3 may comprise a corresponding second touch area (not shown) on the right-hand side of the screen 3, instead or in addition to the second touch area 9, so as to adapt the user interface to a left-handed person. According to other embodiments, the user interface may be realized such that it allows the operator to select the position of the second touch area 9 on the screen 3, for example by allowing the second touch area 9 to be "dragged-and-dropped" on the screen 3 by the operator, or by allowing the operator to indicate a position on the screen 3 for the second touch area 9 prior to use of the breathing apparatus 1.

Preferably, the second touch area 9 is displayed on the contact-sensitive screen 3 in a manner that draws the operator's intention to it when not touched. To this end, the second touch area 9 may be displayed having a first visual appearance when not touched, and a second visual appearance that is different from the first visual appearance when touched. In one embodiment, the second touch area may comprise a blinking element, such as a circle, which stops blinking when the second touch area is touched by the operator. In another embodiment, the second touch area 9 is realized as a button having a first colour which is changed into a second colour when touched. In this way, even a breathing apparatus operator using the proposed user interface for the first time may be guided in the direction of touching the second touch area 9.

Similar solutions as the one described above with reference to Fig. 5 can be used in any of the previously described embodiments in order to help the inexperienced operator to interact with the breathing apparatus 1 using the proposed user interface. In any of the previously described embodiments, the second touch area or areas, generally denoted herein by reference numeral 9, may be displayed having a first visual appearance when not touched, and a second visual appearance when touched by the operator. As mentioned above, the first visual appearance should preferably call for the operator's attention, e.g. by including a blinking element or an element in a prominent colour, while the second visual appearance should indicate to the operator that touching the second touch area is a positive thing, e.g. by making the blinking element stop blinking or changing the colour of the element to a less prominent colour. To further increase the usability and intuitive understanding of the proposed user interface, the first touch area or areas, generally denoted herein by reference numeral 8, may also be displayed having a first visual appearance when the second touch area with which the first touch area is associated (i.e. the second touch area with which the first touch area forms a two-point touch control) is not touched, and a second visual appearance when said second touch area is touched by the operator. Here, the first visual appearance should indicate to the operator that some action has to be taken in order to "activate" the first touch area, i.e. in order to render possible change of a breathing apparatus setting through that first touch area. The second visual appearance should indicate that the first touch area is now "active", i.e. that the breathing apparatus setting can be changed by interaction with the first touch area. To this end, the first visual appearance of the first contact area may include an element, such as a button or a slide-control, which appears semi-transparent and/or in a first, less prominent colour on the contact-sensitive screen 3, whereas the second visual appearance may include the same element appearing opaque and/or in a second, more prominent colour on the screen 3.

In the above description of exemplary views of the user interface of the breathing apparatus according to the invention, the steps involving interpretation and processing of data input by the operator via the contact-sensitive screen 3 are typically performed by the display unit control unit 4 of the breathing apparatus, shown in Fig. 1. Likewise, the steps involving display of information on the contact-sensitive screen based on data input by the operator, or data relating to the operation and/or status of the breathing apparatus, are typically also performed by the display control unit 4. As described with reference to Fig.1, the breathing apparatus may also comprise a central control unit 5 with which the display control unit 4 may be configured to cooperate to control the operation of the breathing apparatus 1 based on data input by the operator via the contact-sensitive screen 3, and to display information relating to the operation of the breathing apparatus 1 on the contact-sensitive screen.

It should also be appreciated that the first touch area may comprise any form of contact-sensitive control means through which the breathing apparatus setting can be changed while simultaneously touching the associated second touch area, and that the control means is not limited to contact-sensitive buttons and slide controls as described with reference to the exemplary embodiments of the invention. For example, the contact-sensitive control means may also be realized in form of a switch that can be placed in any of two or more discrete positions by a finger gesture applied onto the first touch area, or in form of a (digital) rotary knob which may be "rotated" by the operator by a finger gesture applied onto the first touch area.

## Claims

1. A breathing apparatus (1) having a user interface comprising a contact-sensitive screen (3) operable to cause a change in the operation of the breathing apparatus based on touch input from an operator, the contact-sensitive screen comprising a first touch area (8) comprising contact-sensitive control means through which a breathing apparatus setting (*S*) can be changed by the touch of the operator, which breathing apparatus setting (*S*) affects the operation of the breathing apparatus (1) such that the respiratory treatment of a patient connected to the breathing apparatus (1) is adjusted by a change in said breathing apparatus setting (*S*), and at least a second touch area (9) at a distance from said first touch area, wherein the distance between any point of the first touch area (8) and the closest point of the second touch area (9) is at least one centimetre, **characterized in that** the user interface is configured such that the breathing apparatus setting is changed in response to a touch of the contact-sensitive control means of the first touch area (8) only if said second touch area (9) is touched simultaneously.

2. Breathing apparatus (1) according to claim 1, wherein said first touch area (8) comprises contact-sensitive control means in form of a contact-sensitive button, a contact-sensitive slide control, a contact-sensitive switch, or a contact-sensitive rotary knob for changing said breathing apparatus setting (S).

3. Breathing apparatus (1) according to claim 1 or 2, wherein the distance from any point of the first touch area (8) to the closest point of the second touch area (9) is within the range of 1-15 cm in order for said first (8) and second (9) touch areas to form a two-point touch control intended for one-handed operation.

4. Breathing apparatus (1) according to claim 3, wherein the first touch area (8) comprises a contact-sensitive slide control which is elongated, curved and concave in the direction of the second touch area (9) in order for a finger to be moved along the slide control while simultaneously contacting the second touch area with the thumb during a rotary motion of a hand.

5. Breathing apparatus (1) according to claim 3, wherein the first touch area (8) comprises a contact-sensitive slide control, the slide control and the second touch area (9) being substantially straight, elongated areas running in parallel with each other at a distance of 1-10 cm from each other in a direction perpendicular to their longitudinal directions, in order for the operator to change the breathing apparatus setting by sliding two fingers of the same hand along the slide control and the second touch area.

6. Breathing apparatus (1) according to any of the previous claims, wherein said first (8) and/or said second (9) touch area which have to be simultaneously touched in order to change said breathing apparatus setting comprise an element which has a first visual appearance when the second touch area (9) is not touched, and a second visual appearance that is different from said first visual appearance when the second touch area (9) is touched.

7. Breathing apparatus (1) according to any of the preceding claims, wherein said breathing apparatus setting is determinant for the composition of a breathing gas delivered to the patient by the breathing apparatus (1), a target pressure or flow affecting the operation of the breathing apparatus (1), or a mode of ventilation in which the breathing apparatus (1) operates.

8. Breathing apparatus (1) according to claim 1 or 2, wherein the contact-sensitive screen (3) comprises a plurality of second touch areas (9) and a single first touch area (8) comprising a contact-sensitive control means for changing any of a plurality of breathing apparatus settings (*S1-S10*), each of said second touch areas (9) being associated with a particular breathing apparatus setting, wherein any of the plurality of breathing apparatus settings can be changed by simultaneously touching the second touch area associated with the breathing apparatus setting that is to be changed, and the contact-sensitive control means of the first touch area (8).

9. Breathing apparatus (1) according to claim 1 or 2, wherein the contact-sensitive screen (3) comprises a single second touch area (9) and a plurality of first touch areas (8) for changing any of a plurality of breathing apparatus settings (*S1-S6*), each of said first touch areas (8) being associated with a particular breathing apparatus setting and each comprising a contact-sensitive control means trough which said associated breathing apparatus setting can be changed by simultaneously touching the contact-sensitive control means and said single second touch area (9).

10. Breathing apparatus (1) according to claim 1, further comprising a display unit (2) of which the contact-sensitive screen (3) forms a part, and a display control unit (4) configured to receive and process data input by an operator on the contact-sensitive screen (3), and configured to cause information to be displayed on the screen in response thereto, the display control unit (4) being configured to determine whether the first (8) and second touch areas (9) are touched or not, and to effectuate a change in operation of the breathing apparatus (1) in response to a touch of the first touch area (8) only if the second touch area (9) is touched simultaneously.

## Patentansprüche

1. Beatmungsvorrichtung (1) mit einer Benutzergrenzfläche umfassend einen Berührungsbildschirm (3), der betätigt werden kann, um eine Änderung des Betriebs der Beatmungsvorrichtung beruhend auf Berührungseingaben durch einen Betreiber zu bewirken, wobei der Berührungsbildschirm eine erste Berührungszone (8) umfasst, umfassend berührungsempfindliche Steuermittel, durch welche eine Beatmungsvorrichtungeinstellung (S) durch eine Berührung des Betreibers geändert werden kann, welche Beatmungsvorrichtunge-instellung (S) den Betrieb der Beatmungsvorrichtung (1) so beeinflusst, dass die Atembehandlung eines Patienten, der mit der Beatmungsvorrichtung (1) verbunden ist, durch eine Änderung der Beatmungsvorrichtungeinstellung (S) angepasst wird, sowie mindestens eine zweite Berührungszone (9) in einem Abstand von der ersten Berührungszone umfasst, wobei der Abstand zwischen irgendeinem Punkt der ersten Berührungszone (8) und dem nächstgelegenen Punkt der zweiten Berührungszone (9) mindestens 1 Zentimeter ist, **dadurch gekennzeichnet, dass** die Benutzergrenzfläche so ausgeformt ist, dass die Beatmungsvorrichtungeinstellung als Reaktion auf eine Berührung der berührungsempfindlichen Steuermittel der ersten Berührungszone (8) nur dann geändert wird, wenn die zweite Berührungszone (9) gleichzeitig berührt wird.

2. Beatmungsvorrichtung (1) nach Anspruch 1, wobei die erste Berührungszone (8) berührungsempfindliche Steuermittel in Form einer berührungsempfindlichen Taste, einer berührungsempfindlichen Schiebesteuerung, eines berührungsempfindlichen Schalters oder eines berührungsempfindlichen Drehknopfs zum Ändern der Beatmungsvorrichtungeinstellung (S) umfasst.

3. Beatmungsvorrichtung (1) nach Anspruch 1 oder 2, wobei der Abstand von irgendeinem Punkt der ersten Berührungszone (8) zum nächstgelegenen Punkt der zweiten Berührungszone (9) innerhalb eines Bereichs von 1-15 cm liegt, damit die erste (8) und zweite (9) Berührungszone eine Zwei-Punkte-Berührungssteuerung für Einhandbedienung bilden können.

4. Beatmungsvorrichtung (1) nach Anspruch 3, wobei die erste Berührungszone (8) eine berührungsempfindliche Schiebesteuerung umfasst, die in Richtung der zweiten Berührungszone (9) länglich, gekrümmt und konkav ist, damit ein Finger entlang der Schiebesteuerung bewegt werden kann, während bei einer Rotationsbewegung der Hand der Daumen gleichzeitig mit der zweiten Berührungszone in Kontakt ist.

5. Beatmungsvorrichtung (1) nach Anspruch 3, wobei die erste Berührungszone (8) eine berührungsempfindliche Schiebesteuerung umfasst, wobei die Schiebesteuerung und die zweite Berührungszone (9) im Wesentlichen geradlinige, längliche Bereiche sind, die sich parallel zueinander in einem Abstand von 1-10 cm voneinander in einer Richtung senkrecht zu ihren Längsrichtungen erstrecken, damit der Betreiber die Beatmungsvorrichtungeinstellung durch Schieben von zwei Fingern der gleichen Hand entlang der Schiebesteuerung und der zweiten Berührungszone ändern kann.

6. Beatmungsvorrichtung (1) nach einem der vorgehenden Ansprüche, wobei die erste (8) und/oder die zweite (9) Berührungszone, die gleichzeitig berührt werden müssen, um die Beatmungsvorrichtungeinstellung zu ändern, umfassen ein Element, das ein erstes Erscheinungsbild, wenn die zweite Berührungszone (9) nicht berührt wird, und ein zweites Erscheinungsbild, das sich vom ersten Erscheinungsbild unterscheidet, wenn die zweite Berührungszone (9) berührt wird, aufweist.

7. Beatmungsvorrichtung (1) nach einem der vorgehenden Ansprüche, wobei die Beatmungsvorrichtungeinstellung für die Zusammensetzung eines zum Patienten durch die Beatmungsvorrichtung (1) zugeführten Atemgases, einen den Betrieb der Beatmungsvorrichtung (1) beeinflussenden Zieldruck oder Strom, oder einen Beatmungsmodus, in dem die Beatmungsvorrichtung (1) arbeitet, bestimmend ist.

8. Beatmungsvorrichtung (1) nach Anspruch 1 oder 2, wobei der Berührungsbildschirm (3) eine Mehrheit von zweiten Berührungszonen (9) und eine einzige erste Berührungszone (8), umfassend ein berührungsempfindliches Steuermittel zum Ändern von irgendeiner einer Mehrheit von Beatmungsvorrichtungeinstellungen (*S1-S10*), umfasst, wobei jede der zweiten Berührungszonen (9) mit einer bestimmten Beatmungsvorrichtungeinstellung verbunden ist, wobei irgendeine der Mehrheit von Beatmungsvorrichtungeinstellungen durch gleichzeitiges Berühren der zweiten Berührungszone, welche mit der zu ändernden Beatmungsvorrichtungeinstellung verbunden ist, und des berührungsempfindlichen Steuermittels der ersten Berührungszone (8) geändert werden kann.

9. Beatmungsvorrichtung (1) nach Anspruch 1 oder 2, wobei der Berührungsbildschirm (3) eine einzige zweite Berührungszone (9) und eine Mehrheit von ersten Berührungszonen (8) zum Ändern von irgendeiner einer Mehrheit von Beatmungsvorrichtungeinstellungen (*S1-S6*), umfasst, wobei jede der ersten Berührungszonen (8) mit einer bestimmten Beatmungsvorrichtungeinstellung verbunden ist und ein berührungsempfindliches Steuermittel umfasst, durch welches die zugehörige Beatmungsvorrichtungeinstellung durch gleichzeitiges Berühren des berührungsempfindlichen Steuermittels und der einzigen zweiten Berührungszone (9) geändert werden kann.

10. Beatmungsvorrichtung (1) nach Anspruch 1, weiter umfassend eine Anzeigeeinheit (2), von der der Berührungsbildschirm (3) ein Teil ist, und eine Anzeigesteuereinheit (4), die dafür ausgeformt ist, durch einen Betreiber an dem Berührungsbildschirm (3) Dateneingaben zu empfangen und zu behandeln, sowie dafür ausgeformt ist, als Reaktion darauf zu bewirken, dass Information auf dem Schirm angezeigt wird, wobei die Anzeigesteuereinheit (4) dafür ausgeformt ist, zu bestimmen, ob die ersten (8) und zweiten Berührungszonen (9) berührt werden oder nicht, und eine Änderung des Betriebs der Beatmungsvorrichtung (1) als Reaktion einer Berührung der ersten Berührungszone (8) nur dann zu bewirken, wenn die zweite Berührungszone (9) gleichzeitig berührt wird.

## Revendications

1. Dispositif respiratoire (1) avec une interface utilisateur comprenant un écran sensible au contact (3) pouvant être actionné pour provoquer une modification dans l'opération du dispositif respiratoire en fonction de saisie tactile d'un opérateur, l'écran sensible au contact comprenant une première zone de contact tactile (8) comprenant des moyens de commande sensibles au contact par lesquels un fonctionnement du dispositif respiratoire (S) peut être modifié par l'attouchement de l'opérateur, ledit fonctionnement du dispositif respiratoire (S) agit sur l'opération du dispositif respiratoire (1) si bien que le traitement respiratoire d'un patient relié au dispositif respiratoire (1) est ajusté par une modification dans le fonctionnement du dispositif respiratoire (S), et au moins une deuxième zone de contact tactile (9) à une distance de la première zone de contact tactile, la distance entre tout point de la première zone de contact tactile (8) et le point le plus près de la deuxième zone de contact tactile (9) est au moins un centimètre, **caractérisé en ce que** l'interface utilisateur est configurée si bien que le fonctionnement du dispositif respiratoire n'est changé en réponse à un attouchement des moyens de commande sensibles au contact de la première zone de contact tactile (8) que si la deuxième zone de contact tactile (9) est touchée en même temps.

2. Dispositif respiratoire (1) selon la revendication 1, dans lequel ladite première zone de contact tactile (8) comprend des moyens de commande sensibles au contact dans la forme d'un bouton sensible au contact, une commande de glissement sensible au contact, un commutateur sensible au contact ou un bouton rotatif sensible au contact pour modifier ledit fonctionnement du dispositif respiratoire (*S*).

3. Dispositif respiratoire (1) selon la revendication 1 ou 2, dans lequel la distance de tout point de la première zone de contact tactile (8) au point le plus près de la deuxième zone de contact tactile (9) est comprise entre 1-15 cm pour que la première (8) et deuxième (9) zone de contact tactile forment une commande tactile à deux points conçue pour l'opération à une main.

4. Dispositif respiratoire (1) selon la revendication 3, dans lequel la première zone de contact tactile (8) comporte une commande de glissement sensible au contact qui est allongée, incurvée et concave dans la direction de ladite deuxième zone de contact tactile (9) si bien qu'un doigt peut être déplacé le long de la commande de glissement en mettant la deuxième zone de contact tactile simultanément en contact avec le pouce lors d'un mouvement de rotation d'une main.

5. Dispositif respiratoire (1) selon la revendication 3, dans lequel la première zone de contact tactile (8) comporte une commande de glissement sensible au contact, ladite commande de glissement et ladite deuxième zone de contact tactile (9) étant des zones essentiellement rectilignes et allongées s'étendant en parallèle l'une à l'autre à une distance entre elles de 1-10 cm dans une direction perpendiculaire à leurs directions longitudinales de manière à permettre à l'opérateur de modifier le fonctionnement du dispositif respiratoire en faisant glisser deux doigts de la même main le long de la commande de glissière et la deuxième zone de contact tactile.

6. Dispositif respiratoire (1) selon l'une quelconque des revendications précédentes, dans lequel ladite première (8) et/ou ladite deuxième (9) zones de contact tactile qui doivent être touchées en même temps afin de pouvoir changer ledit fonctionnement dudit dispositif respiratoire comprennent un élément qui présente une première apparence visuelle lorsque la deuxième zone de contact tactile (9) n'est pas touchée, et une deuxième apparence visuelle qui est différente de la première apparence visuelle lorsque la deuxième zone de contact tactile (9) est touchée.

7. Dispositif respiratoire (1) selon l'une quelconque des revendications précédentes, dans lequel ledit fonctionnement du dispositif respiratoire est déterminant pour la composition d'un gaz respiratoire délivré au patient par le dispositif respiratoire (1), une pression cible ou un écoulement affectant le fonctionnement du dispositif respiratoire (1), ou un mode de ventilation dans lequel le dispositif respiratoire (1) fonctionne.

8. Dispositif respiratoire (1) selon la revendication 1 ou 2, dans lequel l'écran sensible au contact (3) comprend une pluralité de deuxièmes zones de contact tactile (9) et une seule première zone de contact tactile (8) comprenant un moyen de commande sensible au contact pour modifier l'un quelconque de la pluralité de fonctionnements du dispositif respiratoire (*S1-S10*), chacune desdites deuxièmes zones de contact tactile (9) étant associée avec un fonctionnement particulier du dispositif respiratoire, dans lequel l'un quelconque de la pluralité de fonctionnements du dispositif respiratoire peut être modifié par un attouchement simultané de la deuxième zone de contact tactile associée avec le fonctionnement du dispositif respiratoire à modifier, et du moyen de commande sensible au contact de la première zone de contact tactile (8).

9. Dispositif respiratoire (1) selon la revendication 1 ou 2, dans lequel l'écran sensible au contact (3) comprend une seule zone de contact tactile (9) et une pluralité de premières zones de contact tactile (8) pour modifier l'un quelconque de la pluralité de fonctionnements du dispositif respiratoire (*S1-S6*), chacune desdites premières zones de contact tactile (8) étant associée avec un fonctionnement particulier du dispositif respiratoire, et chacune comprenant un moyen de commande sensible au contact par lequel ledit fonctionnement du dispositif respiratoire associé peut être modifié par un attouchement simultané du moyen de commande sensible au contact et de la seule deuxième zone de contact tactile (9).

10. Dispositif respiratoire (1) selon la revendication 1, comprenant en outre une unité d'affichage (2) dont l'écran sensible au contact (3) fait partie, et une unité de commande d'affichage (4) configurée pour recevoir et saisir des entrées de données par un opérateur sur l'écran sensible au contact (3), et configurée pour faire afficher des informations sur l'écran en réponse à celle-ci, l'unité de commande d'affichage (4) étant configurée pour déterminer si la première (8) et la deuxième (9) zones de contact tactile sont touchées ou non, et pour effectuer une modification dans le fonctionnement du dispositif respiratoire (1) en réponse à un attouchement de la première zone de contact tactile (8) uniquement si la deuxième zone de contact tactile (9) est touchée simultanément.
